# EUROPEAN PATENT SPECIFICATION

(11) **EP 3 157 494 B1**
(45) Date of publication and mention of the grant of the patent: **24.10.2018**
(21) Application number: 15730166.4
(22) Date of filing: 19.06.2015
(51) Int. Cl.: A61K 8/03, A61K 8/92, A61K 8/891, A61K 8/39, A61Q 5/12, A61K 8/34, A61K 8/31

(54) **MULTI-PHASE ANHYDROUS COMPOSITION COMPRISING AT LEAST ONE OIL, AT LEAST ONE POLYOL AND NON IONIC SURFACTANTS, AND COSMETIC PROCESS THEREOF**
MEHRPHASIGE WASSERFREIE ZUSAMMENSETZUNG MIT MINDESTENS EINEM ÖL, MINDESTENS EINEM POLYOL UND NICHTIONISCHEN TENSIDEN UND KOSMETISCHES VERFAHREN DARAUS
COMPOSITION ANHYDRE À PHASES MULTIPLES COMPRENANT AU MOINS UNE HUILE, AU MOINS UN POLYOL ET DES SURFACTANTS NON IONIQUES, ET PROCÉDÉ COSMÉTIQUE ASSOCIÉ

(30) Priority: 19.06.2014 IN 1649DE2014
(43) Date of publication of application: 26.04.2017
(73) Proprietor: L'Oréal, 75008 Paris (FR)
(72) Inventor: DE BONI, Maxime, Chemnur Maharashtra 400071 (IN)
(74) Representative: Casalonga
(86) International application number: PCT/EP2015/063875
(87) International publication number: WO 2015/193497

(56) References cited:
- EP-A1- 1 593 364
- EP-A1- 2 438 905
- WO-A2-2013/092293
- US-A1- 2003 143 178

## Description

The present invention relates to an anhydrous composition for treating keratin materials, such as keratin fibres, in particular human keratin fibres such as the hair, comprising at least one oily phase (A), at least one anhydrous non-oily phase (B), at least one non ionic surfactant (i) having an HLB higher than 9 and at least one non ionic surfactant (ii) having an HLB less than or equal to 9 in a specific ratio, wherein said phases (A) and (B) are visually distinct from each other.

The invention also concerns a process for treating keratin materials, such as keratin fibres, in particular human keratin fibres such as the hair, and a use for hair care employing the said anhydrous composition.

Many people are unsatisfied with the way their hair looks, and have difficulty in styling it. Hair is generally damaged and embrittled by the action of external atmospheric agents such as light and bad weather, and also by mechanical or chemical treatments, such as brushing, combing, dyeing, bleaching, permanent-waving and/or relaxing. Hence, hair is damaged by these various factors and may over time become dry, coarse or dull, and less manageable.

Thus, to overcome these drawbacks, it is common practice to resort to hair care products using compositions that condition the hair appropriately, giving it satisfactory cosmetic properties, especially in terms of smoothness, sheen, softness, suppleness, lightness, a natural feel and good disentangling properties.

These hair care compositions may be, for example, conditioning shampoos, hair conditioners, masks or sera, and may be in the form of gels, hair lotions or care creams that are more or less thick.

Consumers seek more and more hair care compositions that are not only able to confer good cosmetic properties to the hair but also have an overall pleasant aspect.

Unfortunately most of the available hair care compositions often fail to have an aesthetically pleasing appearance because of their texture which can be white and creamy. In other words, there is a need to develop hair care compositions having an attractive aspect in order to stimulate visually potential consumers.

Furthermore, oils are commonly used as conditioning agents in hair care cosmetic compositions in order to provide conditioning effects for hair, in particular to provide softness and brightness to dry or sensitive hair. These compositions can be used before or after the application of shampoos. In tropical countries such as India, these compositions are preferably used before the application of shampoos in order to nourish the hair.

For example, the patent application WO 2013/092293 describes the use of a unique blend of vegetable oil and mineral oil along with silicones in order to provide smooth and manageable hair feel, without conferring a sensation of stickiness on hair and hand.

In the same manner, the document US 6989148 describes the use of a water immiscible solvent with one or more surfactants, which on dilution form a micro- or macro-emulsion in situ to deliver agents otherwise reactive when in prolonged contact with water. These agents are capable of conditioning the hair.

Furthermore, the patent US 4438095 discloses a non-detergent, non-foaming cosmetic composition for conditioning the hair consisting essentially of two separate liquid phases, one of which being aqueous and in which at least one cationic polymer is dissolved.

However, these cosmetic compositions are often difficult to be rinsed off with water which implies that it may be difficult to remove such compositions from the hair.

Moreover, these cosmetic compositions still give unsatisfied cosmetic properties to the hair, namely in terms of smoothness, lightness, stickiness and manageability.

Hence since such cosmetic compositions can be difficult to be removed from the hair they often leave a sticky feeling to the hair.

Therefore, there is a real need to develop compositions, for example cosmetic compositions for treating keratin fibres, in particular human keratin fibres such as the hair that do not have the combination of drawbacks described above, i.e. which can be easily rinsed off and can improve the cosmetic properties of said fibers, namely by affording them smoothness, lightness and manageability, while having an attractive aspect in order to stimulate visually potential consumers.

The Applicant has discovered, surprisingly, that it is possible to formulate anhydrous compositions having at least two visually distinctive phases, wherein one of the phases is an oily phase containing at least one oil and the other distinct phase is an anhydrous non-oily phase containing at least one polyol, and two different non ionic surfactants in a specific ratio as described below that are capable to lead to the desired properties.

In particular, it has been found that such anhydrous compositions are easily rinsed off, namely with water, which means that they can be easily removed from hair, especially before the application of shampoos.

Furthermore, such anhydrous compositions can contain both hydrophobic and hydrophilic active agents which could be helpful for conditioning the hair. Thus the anhydrous compositions can constitute a good medium for water soluble active agents.

The anhydrous compositions of the present invention also improve the cosmetic properties conferred to the hair, namely by affording it smoothness, softness, lightness and more manageability while at the same time leaving it less sticky. In particular, such compositions leave improved sensory benefits both on hair and hand.

In other words, the anhydrous compositions are able to provide improved conditioning to the hair and self-emulsification when diluted with water.

The anhydrous compositions according to the invention may have multiple liquid phases, preferably two liquid phases. Each liquid phase can provide any independent visual effect. Thus, the multi liquid phases confer unique appearances to the anhydrous compositions of the present invention. In other words, the anhydrous compositions have an overall attractive aspect to the consumers.

On the other hand, the multiple liquid phases can disappear when mixing them by, for example, shaking the anhydrous composition of the invention to form a uniform phase. Thus for example, if the refractive indexes of the multiple liquid phases are similar to each other, it is possible for the uniform phase to be transparent. The uniform phase can separate into the multiple liquid phases again by leaving it, without any shear force, in a certain period of time such as from few minutes to several hours.

The present invention relates especially to an anhydrous composition comprising:
- at least one oil phase (A) comprising at least one oil,
- at least one anhydrous non-oil phase (B) comprising at least one polyol, in an amount ranging from 60 to 95% by weight relative to the total weight of the non-oil phase (B),
- at least one non ionic surfactant (i) having an HLB higher than 9,
- at least one non ionic surfactant (ii) having an HLB less than or equal to 9 in a quantity of at least 0.2% by weight relative to the weight of the anhydrous composition;
wherein the non ionic surfactant (i) and the non ionic surfactant (ii) are present in the composition in a weight ratio of from 5.5 to 12.5, and
wherein said phases (A) and (B) are visually distinctive from each other.

The terms "visually distinctive phases" according to the present invention mean that theses phases may be distinguished from each other to the naked eye by a human being, contrary to phases forming emulsions or dispersions of homogeneous particles. More particularly, none of the phases is in the form of globules.

Preferably, the anhydrous composition comprises two phases (A) and (B) that are visually distinctive from each other.

As previously mentioned, the anhydrous composition of the present invention is optically clear.

According to the present invention, the terms "optically clear" means than turbidity is less than 300 NTU (Nephelometric Turbidity Units), which is the unit of measure for the turbidity or haze of a liquid. More preferably, the optically clear anhydrous compositions of the invention have a turbidity ranging from 0,05 to 100 NTU, better from 1 to 80 NTU.

For the purposes of the present invention, the term "anhydrous composition" means a composition containing only a small amount of water, preferably no water (0%). Thus the amount of water may be 2% by weight or less, preferably 1.5% by weight or less, more preferably 1% by weight or less relative to the total weight of the said composition. It is particularly preferable that the anhydrous composition according to the present invention contains no water.

The present invention also relates to a process for cosmetically treating keratin materials, such as keratin fibres, in particular human keratin fibres such as the hair, comprising the application to the said materials of the anhydrous composition according to the invention.

Other subjects and characteristics, aspects and advantages of the invention will emerge even more clearly on reading the description and the example that follows.

In the text hereinbelow, and unless otherwise indicated, the limits of a range of values are included in that range, in particular in the expressions "between" and "ranging from ... to ...".

Moreover, the expression "at least one" used in the present description is equivalent to the expression "one or more".

### Oily phase (A)

The anhydrous composition comprises at least one oil phase (A) including at least one oil. Two or more oils may be used in combination. Thus, a single type of oil or a combination of different type of oils may be used.

For the purposes of the present invention, the term "oil" refers to a fatty substance that is liquid at ambient temperature (25°C) and at atmospheric pressure (760 mmHg, i.e. 1.013× 10⁵ Pa).

The term "fatty substance" means an organic compound that is insoluble in water at ambient temperature (25°C) and at atmospheric pressure (760 mmHg, i.e. 1.013× 10⁵ Pa), i.e. with a weight solubility of less than 5%, preferably less than 1% and even more preferentially less than 0.1%. They have in their structure at least one hydrocarbon chain containing at least six carbon atoms or at least one sequence of at least two siloxane groups. In addition, the fatty substances are generally soluble in organic solvents under the same temperature and pressure conditions, for instance chloroform, ethanol, benzene, liquid petroleum jelly or decamethyl cyclopentasiloxane.

Preferably, the oil phase (A) of the anhydrous composition of the present invention is liquid at ambient temperature (25°C) under atmospheric pressure (760 mmHg, i.e. 1.013× 10⁵ Pa).

Preferably, the oil used in the present invention does not contain a polyalkylenated or polygycerolated group or a salified carboxylic group.

The oil may be selected from the group consisting of oils of animal or plant origin, synthetic glycerides, esters of fatty alcohols and/or fatty acids other than animal or plant oils and synthetic glycerides, fatty alcohols, non salified fatty acids, silicone oils and aliphatic hydrocarbons. These fatty materials may be volatile or nonvolatile.

Preferably, the oil is selected from aliphatic hydrocarbons, plant oils, fatty alcohols, esters of fatty alcohols and/or fatty acids other than animal or plant oils and synthetic glycerides, or mixtures thereof. More preferably the oil is selected from the group consisting of aliphatic hydrocarbons, in particular mineral oils.

As examples of aliphatic hydrocarbons, mention may be made of, for example, linear or branched hydrocarbons such as mineral oils (e.g., liquid paraffin), paraffin, vaseline or petrolatum, naphthalenes, and the like; hydrogenated polyisobutene, isoeicosan, polydecenes, hydrogenated polyisobutenes such as Parleam, and decene/butene copolymer; and mixtures thereof.

As examples of other aliphatic hydrocarbons, mention may also be made of linear or branched, or possibly cyclic C₆-C₁₆ lower alkanes. Examples that may be mentioned include hexane, undecane, dodecane, tridecane and isoparaffins such as isohexadecane and isodecane.

As example of synthetic glycerides, mention may be made of, for instance, caprylic/capric acid triglycerides, for instance those sold by the company Stéarineries Dubois or those sold under the names Miglyol® 810, 812 and 818 by the company Dynamit Nobel.

As examples of silicone oils, mention may be made of, for example, linear organopolysiloxanes such as dimethylpolysiloxane, methylphenylpolysiloxane, methylhydrogenpolysiloxane, and the like; cyclic organopolysiloxanes such as octamethylcyclotetrasiloxane, decamethylcyclopentasiloxane, dodecamethylcyclohexasiloxane, and the like; and mixtures thereof.

As examples of plant oils, mention may be made of, for example, linseed oil, camellia oil, macadamia nut oil, sunflower oil, apricot oil, soybean oil, arara oil, hazelnut oil, corn oil, mink oil, olive oil, avocado oil, sasanqua oil, castor oil, safflower oil, jojoba oil, sunflower oil, almond oil, grapeseed oil, sesame oil, soybean oil, peanut oil, and mixtures thereof.

As examples of animal oils, mention may be made of, for example, squalene, perhydrosqualene and squalane.

As examples of the esters of fatty alcohols and/or of fatty acids, which are advantageously different from the animal or plant oils as well as the synthetic glycerides mentioned above, mention may be made especially of esters of saturated or unsaturated, linear or branched C₁-C₂₆ aliphatic mono- or polyacids and of saturated or unsaturated, linear or branched C₁-C₂₆ aliphatic mono- or polyalcohols, the total carbon number of the esters being greater than or equal to 10.

Among the monoesters, mention may be made of dihydroabietyl behenate; octyldodecyl behenate; isocetyl behenate; cetyl lactate; C₁₂-C₁₅ alkyl lactate; isostearyl lactate; lauryl lactate; linoleyl lactate; oleyl lactate; (iso)stearyl octanoate; isocetyl octanoate; octyl octanoate; cetyl octanoate; decyl oleate; isocetyl isostearate; isocetyl laurate; isocetyl stearate; isodecyl octanoate; isodecyl oleate; isononyl isononanoate; isostearyl palmitate; methylacetyl ricinoleate; myristyl stearate; octyl isononanoate; 2-ethylhexyl isononate; octyl palmitate; octyl pelargonate; octyl stearate; octyldodecyl erucate; oleyl erucate; ethyl and isopropyl palmitates, 2-ethylhexyl palmitate, 2-octyldecyl palmitate, alkyl myristates such as isopropyl, butyl, cetyl, 2-octyldodecyl, myristyl or stearyl myristate, hexyl stearate, butyl stearate, isobutyl stearate; dioctyl malate, hexyl laurate, 2-hexyldecyl laurate.

Still within the context of this variant, esters of C₄-C₂₂ dicarboxylic or tricarboxylic acids and of C₁-C₂₂ alcohols and esters of mono-, di- or tricarboxylic acids and of C₂-C₂₆ di-, tri-, tetra- or pentahydroxy alcohols may also be used.

The following may especially be mentioned: diethyl sebacate; diisopropyl sebacate; diisopropyl adipate; di-n-propyl adipate; dioctyl adipate; diisostearyl adipate; dioctyl maleate; glyceryl undecylenate; octyldodecyl stearoyl stearate; pentaerythrityl monoricinoleate; pentaerythrityl tetraisononanoate; pentaerythrityl tetrapelargonate; pentaerythrityl tetraisostearate; pentaerythrityl tetraoctanoate; propylene glycol dicaprylate; propylene glycol dicaprate; tridecyl erucate; triisopropyl citrate; triisostearyl citrate; glyceryl trilactate; glyceryl trioctanoate; trioctyldodecyl citrate; trioleyl citrate; propylene glycol dioctanoate; neopentyl glycol diheptanoate; diethylene glycol diisononanoate; and polyethylene glycol distearates.

Among the esters mentioned above, it is preferred to use ethyl, isopropyl, myristyl, cetyl or stearyl palmitate, 2-ethylhexyl palmitate, 2-octyldecyl palmitate, alkyl myristates such as isopropyl, butyl, cetyl or 2-octyldodecyl myristate, hexyl stearate, butyl stearate, isobutyl stearate; dioctyl malate, hexyl laurate, 2-hexyldecyl laurate, isononyl isononanoate or cetyl octanoate.

The oil phase may also comprise, as fatty ester, sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which contain at least 4 carbon atoms. These sugars may be monosaccharides, oligosaccharides or polysaccharides.

Examples of suitable sugars that may be mentioned include sucrose (or saccharose), glucose, galactose, ribose, fructose, maltose, mannose, arabinose, xylose and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may comprise one to three conjugated or non-conjugated carbon-carbon double bonds.

The esters according to this variant may also be chosen from mono-, di-, tri-, tetraesters and polyesters, and mixtures thereof.

These esters may be chosen, for example, from oleates, laurates, palmitates, myristates, behenates, cocoates, stearates, linoleates, linolenates, caprates and arachidonates, or mixtures thereof such as, especially, oleo-palmitate, oleo-stearate and palmito-stearate mixed esters.

It is more particularly preferred to use monoesters and diesters and especially sucrose, glucose or methylglucose mono- or dioleates, stearates, behenates, oleopalmitates, linoleates, linolenates and oleostearates.

An example that may be mentioned is the product sold under the name Glucate® DO by the company Amerchol, which is a methylglucose dioleate.

Examples of esters or mixtures of esters of sugar and of fatty acid that may also be mentioned include:
- the products sold under the names F160, F140, F110, F90, F70 and SL40 by the company Crodesta, respectively denoting sucrose palmitostearates formed from 73% monoester and 27% diester and triester, from 61% monoester and 39% diester, triester and tetraester, from 52% monoester and 48% diester, triester and tetraester, from 45% monoester and 55% diester, triester and tetraester, from 39% monoester and 61% diester, triester and tetraester, and sucrose monolaurate;
- the products sold under the name Ryoto Sugar Esters, for example referenced B370 and corresponding to sucrose behenate formed from 20% monoester and 80% di- triester-polyester;
- the sucrose mono-dipalmito-stearate sold by the company Goldschmidt under the name Tegosoft® PSE.

The oil phase may include be at least one fatty acid. Two or more fatty acids may be used. The fatty acids should be in acidic form (i.e., unsalified, to avoid soaps) and may be saturated or unsaturated and contain from 6 to 30 carbon atoms and in particular from 9 to 30 carbon atoms, which is optionally substituted, in particular with one or more hydroxyl groups (in particular 1 to 4). If they are unsaturated, these compounds may comprise one to three conjugated or non-conjugated carbon-carbon double bonds. They are more particularly chosen from myristic acid, palmitic acid, stearic acid, behenic acid, oleic acid, linoleic acid, linolenic acid and isostearic acid. Preferably the fatty material is not a fatty acid.

The oil phase may include at least one fatty alcohol, and two or more fatty alcohols may be used.

The term "fatty alcohol" here means any saturated or unsaturated, linear or branched C₈-C₃₀ fatty alcohol, which is optionally substituted, in particular with one or more hydroxyl groups (in particular 1 to 4). If they are unsaturated, these compounds may comprise one to three conjugated or non-conjugated carbon-carbon double bonds. Preferably fatty alcohols are unsaturated and/or branched.

Among the C₈-C₃₀ fatty alcohols, C₁₂-C₂₂ fatty alcohols, for example, are used. Mention may be made among these of, isostearyl alcohol, oleyl alcohol, linoleyl alcohol, undecylenyl alcohol, palmitoleyl alcohol, linolenyl alcohol, erucyl alcohol, and mixtures thereof. In one embodiment, cetyl alcohol, stearyl alcohol or a mixture thereof (e.g., cetearyl alcohol), as well as myristyl alcohol, can be used as a solid fatty material. In one embodiment, isostearyl alcohol can be used as a liquid fatty material.

Preferably, the oils used in phase (A) are chosen from aliphatic hydrocarbons, plant oils, silicone oils and esters of fatty alcohols and/or fatty acids other than animal or plant oils, and their mixtures.

More preferably, the oils used in phase (A) are chosen from aliphatic hydrocarbons, plant oils and their mixture.

According to a preferred embodiment, the oily phase (A) comprises at least one aliphatic hydrocarbon based oil and at least one plant oil.

According to this embodiment, the oily phase (A) may further comprise at least one fatty ester such as C₂-C₄ alkyl myristates, in particular isopropyl myristate and at least one silicone oil.

The amount of the oil phase (A) may range from 10 to 90 % by weight, preferably from 20 to 80% by weight, and more preferably from 30 to 70% by weight, relative to the total weight of the composition.

The amount of oil(s) in the composition may range from 30 to 85% by weight, preferably 40 to 80% by weight, and more preferably from 50 to 70% by weight, relative to the total weight of the composition.

The amount of oil(s) in the oil phase (A) may range from 50 to 100% by weight, preferably 60 to 95% by weight, and more preferably from 70 to 90% by weight, relative to the total weight of the oil phase.

In a preferred embodiment, the oily phase (A) comprises at least one aliphatic hydrocarbon based oil in an amount ranging from 40% to 70% by weight and at least one plant oil in an amount ranging from 10% to 30% by weight relative to the weight of the oily phase (A) and optionally at least one oil chosen from the silicone oils and the fatty esters in an amount less than 8% by weight relative to the weight of the oily phase (A).

The oil phase may include at least one colorant, but it is preferable that the oil phase be transparent or translucent.

The oil phase may include at least one lipophilic compound such as oil-soluble organic or inorganic compounds (e.g., some types of amino acids). The amount of the lipophilic compound is 50% by weight or less relative to the total weight of the oil phase.

### Anhydrous non-oil phase (B)

As previously mentioned, the anhydrous composition of the present invention also comprises at least one anhydrous non-oil phase (B) containing at least one polyol.

Preferably, the anhydrous non-oil phase (B) is liquid at ambient temperature (25°C) under atmospheric pressure (760 mmHg, i.e. 1.013× 10⁵ Pa).

For the purposes of the present invention, the terms "anhydrous non-oil phase (B)" refer to a phase having a water content of less than 2% by weight, preferably less than 1.5% by weight and even more preferably less than 1% by weight relative to the weight of said phase, most preferably containing no water (0%).

Furthermore, the terms "anhydrous non-oil phase (B)" also refer to a phase having an oil content of less than 2% by weight, preferably less than 1.5% by weight and even more preferably less than 1% by weight relative to the weight of the said phase, most preferably containing no oil (0%).

For the purposes of the present invention, the polyol used in phase (B) is preferably liquid at 25°C and 1 atm.; the polyol according to the invention is different from the hereafter non ionic surfactants.

Preferably, the polyols used in the phase (B) have the corresponding formula (I): 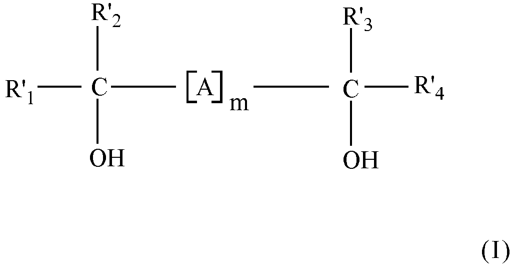 in which R'₁, R'₂, R'₃ and R'₄ denote, independently of each other, a hydrogen atom, a C₁-C₆ alkyl radical or a C₁-C₆ mono- or polyhydroxyalkyl radical,

A denotes a linear or branched alkylene radical containing from 1 to 18 carbon atoms, this radical comprising from 0 to 9 oxygen atoms,
m denotes 0 or 1.

A first group of preferred polyols consists of the polyols of formula V for which m = 0, such as glycerin, 1,2,3-propanetriol, pinacol (2,3-dimethyl-2,3-butanediol), 1,2,3-butanetriol, 2,3-butanediol and sorbitol.

A second group of preferred polyols consists of the polyols of formula V for which m = 1 and R'₁, R'₂, R'₃ and R'4 denote, independently of each other, a hydrogen atom or a C₁-C₆ alkyl radical. Among these polyols, polyethylene glycols, for instance the product known as PEG-8 or PEG-400 in the CTFA publication (International Cosmetic Ingredient Dictionary, 7th edition), are particularly preferred.

A third group of preferred polyols consists of the polyols of formula V for which m = 1 and R'₁, R'₂, R'₃ and R'₄ denote, independently of each other, a hydrogen atom or a C₁-C₆ alkyl radical, and whose molecular weight is less than 200. Among these polyols, 3-methyl-1,3,5-pentanetriol, 1,2,4-butanetriol, 1,5-pentanediol, 2-methyl-1,3-propanediol, 1,3-butanediol, 3-methyl-1,5-pentanediol, neopentyl glycol (2,2-dimethyl-1,3-propanediol), isoprene glycol (3-methyl-1,3-butanediol) and hexylene glycol (2-methyl-2,4-pentanediol) are preferably used, and even more preferably hexylene glycol, neopentyl glycol and 3-methyl-1,5-pentanediol.

Preferably, the polyols used in the phase (B) are chosen from glycerin, propylene glycol and polyethylene glycols having from 2 to 9 ethylene oxide units (OE) such as PEG-8.

In a preferred embodiment, the polyols used in the phase (B) corresponds to formula (V) wherein m = 1.

More preferably, the polyols used in the anhydrous non-oil phase (B) are chosen from polyethylene glycols and even more preferably contain PEG-8.

The polyols may be present in the anhydrous composition in an amount preferably ranging from 10% to 40% by weight, better still from 15% to 35% by weight and even more preferably from 20% to 30% by weight relative to the weight of the composition.

The polyols may be present in the non oil phase in an amount ranging from 60% to 95% by weight, better still from 70% to 95% by weight and even more preferably from 75% to 90% by weight relative to the weight of the non oil phase.

The amount of the anhydrous non-oil phase (B) may range from 10 to 90% by weight, preferably from 20 to 80% by weight, and more preferably from 30 to 70% by weight, relative to the total weight of the composition.

### Non ionic surfactant (i) having an HLB higher than 9

As previously mentioned, the anhydrous composition of the present invention also comprises at least one non ionic surfactant (i) having an HLB higher than 9.

The terms "HLB higher than 9" mean a non ionic surfactant (i) having at 25°C an HLB (hydrophilic-lipophilic balance) value under Griffin higher than 9.

The HLB value according to Griffin is defined in J. Soc. Cosm. Chem. 1954 (volume 5), pp. 249-256.

The non ionic surfactants (i) having an HLB higher than 9 are preferentially chosen from non ionic oxyethylenated surfactants having a number of ethylene oxide higher than 5.

In particular, the non ionic surfactant (i) having an HLB higher than 9 is selected from polyethoxylated fatty monoalcohols in C₈-C₃₀ or polyethoxylated fatty esters in C₈-C₃₀, the number of ethylene oxide being higher than 5, preferably from 5 to 30, preferably from 6 to 25.

Most preferably, the non ionic surfactant (i) is selected from polyethoxylated fatty esters in C₈-C₃₀, the number of ethylene oxide being higher than 5, preferably from 5-30, most preferably from 6-25.

Preferably, the non ionic surfactant (i) may be chosen from oxyethylenated lauryl alcohol containing 12 moles of ethylene oxide (HLB = 14.5), oxyethylenated oleyl alcohol containing 30 moles of ethylene oxide (HLB = 16.6), oxyethylenated oleyl alcohol containing 10 moles of ethylene oxide (HLB = 12.4), polyethylene glycol-7 (PEG-7) glyceryl cocoate (HLB = 11.0) and polyethylene glycol-6 caprylic capric triglyceride (HLB = 13.2).

The non ionic surfactant (i) may be present in phase(s) (A) and/or (B) of the anhydrous composition of the present invention and preferably is present in the anhydrous non-oil phase (B) of the anhydrous composition.

The non ionic surfactant (i) is preferably present in an amount ranging from 1 to 20% by weight, better still from 1.5 to 15% by weight, even better from 2 to 10% by weight relative to the weight of the anhydrous composition.

### Non ionic surfactant (ii) having an HLB less than or equal to 9

The anhydrous composition of the invention further comprises at least one non ionic surfactant (ii) having an HLB less than or equal to 9.

The terms "HLB less than or equal to 9" mean a non ionic surfactant (ii) having at 25°C an HLB (hydrophilic-lipophilic balance) under Griffin less than or equal to 9.

The HLB value according to Griffin is defined in J. Soc. Cosm. Chem. 1954 (volume 5), pp. 249-256.

The non ionic surfactants (ii) having an HLB less than or equal to 9 are preferentially chosen from non ionic oxyethylenated surfactants having a number of ethylene oxide lower than 5.

In particular, the non ionic surfactant having an HLB less than or equal to 9 is selected from polyethoxylated fatty monoalcohols in C₈-C₃₀ or polyethoxylated fatty esters in C₈-C₃₀, the number of ethylene oxide being lower than or equal to 5, preferably from 1 to 5.

Most preferably, the non ionic surfactant having an HLB less than or equal to 9 (ii) is selected from polyethoxylated fatty monoalcohols in C₈-C₃₀, the number of ethylene oxide being lower than or equal to 5, preferably from 1 to 5.

Preferably, the non ionic surfactants (ii) may be chosen from oxyethylenated decyl alcohol containing 3.5 mole of ethylene oxide (HLB = 8.5), oxyethylenated isostearyl alcohol containing 2 moles of ethylene oxide (HLB = 6.5), oxyethylenated lauryl alcohol containing 3 moles of ethylene oxide (HLB = 8.0), oxyethylenated C₅₀ primary alcohol containing 4 moles of ethylene oxide (HLB = 4).

The non ionic surfactant having an HLB less than or equal to 9 may be present in phase(s) (A) and/or (B) and is preferably present in the anhydrous non-oil phase (B).

The non ionic surfactant having an HLB less than or equal to 9 is present in the composition in an amount of at least 0.2% by weight, preferably in an amount ranging from 0.2 to 1.5% by weight, better 0.25 to 1.3% by weight and even better 0.25 to 1.2% by weight relative to the total weight of the anhydrous composition.

According to the present invention, the non ionic surfactant(s) (i) and the non ionic surfactant(s) (ii) are present in the composition in a weight ratio (i):(ii) of from 5,5 to 12,5, better from 6 to 12, even better from 6 to 10. In a preferred embodiment, the anhydrous composition of the present invention comprises:
- at least one oil phase (A) comprising at least one oil chosen from aliphatic hydrocarbons, plant oils, silicone oils and esters of fatty alcohols and/or fatty acids other than animal or plant oils, and their mixtures, more preferably at least one aliphatic hydrocarbon and at least one plant oil,
- at least one anhydrous non-oil phase (B) comprising at least one polyol chosen from polyethylene glycol having 2 to 9 OE,
- at least one non ionic surfactant (i) having an HLB higher than 9,
- at least one ionic surfactant (ii) having an HLB less than or equal to 9 in an amount of at least 0.2% by weight relative to the total weight of the anhydrous composition.

According to this embodiment, the non ionic surfactant (i) and the non ionic surfactant (ii) are present in the composition in a weight ratio of from 5.5 to 12.5.

According to this embodiment, the polyols may be present in the anhydrous composition in an amount preferably ranging from 10% to 40% by weight relative to the weight of the composition. Preferably, the polyols may be present in the anhydrous composition in an amount ranging from 15% to 35% by weight and even more preferably from 20% to 30% by weight relative to the weight of the composition.

According this embodiment, the non ionic surfactant (i) is preferably chosen from polyethoxylated fatty esters in C₈-C₃₀, the number of ethylene oxide being higher than 5, preferably from 5 to 30, most preferably from 6 to 25 and non ionic surfactant (ii) is preferably chosen from polyethoxylated fatty monoalcohols in C₈-C₃₀, the number of ethylene oxide being lower than or equal to 5, preferably from 1 to 5.

According to this embodiment, the ratio between phase (A) and phase (B) is preferably ranging from 10:90 to 90:10, more preferably from 25:75 to 75:25.

According to the present invention, the oil phase (A) and the anhydrous non oil phase (B) can be directly in contact to each other. Typically, the above two phases may be packaged in a single container.

It is preferable that the oil phase (A) and the anhydrous non oil phase (B) in the anhydrous cosmetic composition according to the present invention can be spontaneously separated from each other. Preferably, the at least two phases (A) and (B) are visually distinct after coming in a state of rest. More preferably, the two phases can be separated. This phase separation can visually stimulate the users of the anhydrous cosmetic composition according to the present invention. The phase separation of the anhydrous cosmetic composition according to the present invention can be caused by leaving it without any shear force for a certain period of time such as 10 minutes to 24 hours.

It is preferable that the anhydrous cosmetic composition according to the present invention can form a homogeneous phase when being mixed by, for example, shaking the composition by hand or diluted with water. It is preferable that the homogeneous phase be in the lamellar form, and viscous such that it does not drip off.

The viscosity of the anhydrous cosmetic composition according to the present invention is not particularly limited. The viscosity can be measured at 25°C with viscosimeters or rheometers preferably with cone-plane geometry. Preferably, the viscosity of the anhydrous cosmetic composition according to the present invention can range, for example, from 1 to 2000 Pa.s, and preferably from 1 to 1000 Pa.s at 25°C and 1s⁻¹.

The anhydrous cosmetic composition according to the present invention may also comprise an effective amount of cosmetic additives such as anionic, cationic or amphoteric surfactants, thickeners, sequestering agents, UV screening agents, preserving agents, vitamins or provitamins, opacifiers, fragrances, plant extracts, humectants, waxes, fillers, colouring materials, antioxydants, proteins, and so on.

If any of the oil phase (A) and the anhydrous non oil phase (B) is colored, the anhydrous cosmetic composition according to the present invention may be more visually attractive for users.

The anhydrous cosmetic composition according to the present invention can be prepared by mixing the above essential or optional components by using a conventional mixing means such as a mixer and a homogenizer, preferably in a weight ratio 50/50.

The anhydrous cosmetic composition according to the present invention can be used as cosmetics for hair, such as hair rinse-off or leave on products (e.g., shampoos and conditioners).

The anhydrous cosmetic composition may also comprise one or more additives chosen from amino acids, proteins, vitamins, non oil soluble plant extracts, glycerol, fragrances, dyes and alkaline agents.

### Cosmetic process and use

The anhydrous composition according to the present invention can be used in a cosmetic process for keratin substance comprising the step of applying the anhydrous composition according to the present invention to keratin materials, such as keratin fibres, in particular human keratin fibres such as the hair.

The keratin fibres can be in a dry state or in a wet state before application of the cosmetic composition according to the present invention. The application of the composition according to the invention to the keratin fibres may or may not be followed by rinsing the keratin substance. Before rinsing, the anhydrous composition according to the present invention can be left in contact with the keratin substance, for example, from 30 seconds to 30 minutes.

It is preferable that the cosmetic process for keratin materials according to the present invention comprise the step of mixing the oil phase (A) and the anhydrous non-oil phase (B) of the anhydrous cosmetic composition, for example by hand, before the step of applying the anhydrous composition to the keratin materials.

By mixing the anhydrous composition according to the present invention, a homogeneous phase is formed. Since the homogeneous phase includes oil(s), polyol(s) and nonionic surfactant(s) equally, the cosmetic effects provided by these ingredients can be provided uniformly to the keratin substance, which will result in good balance of cosmetic effects.

Since the homogeneous phase is not in the form of an emulsion, the oil and the polyol can directly contact the keratin fibres, and therefore, the anhydrous composition according to the present invention can provide superior conditioning effects. The composition is capable of forming a layer on keratin fibres which can inhibit the evaporation of water from the keratin fibres, while it can solubilize hydrophobic or hydrophilic substances).

The invention also relates to the use of the anhydrous composition according to the invention for the conditioning of keratin fibres, in particular human keratin fibres such as the hair, preferably used before the application of a shampoo.

### EXAMPLES

### Example 1:

### I. Tested compositions

The following compositions are prepared with the ingredients listed in the table below. The quantities are expressed in grams (g) of active matter (MA).

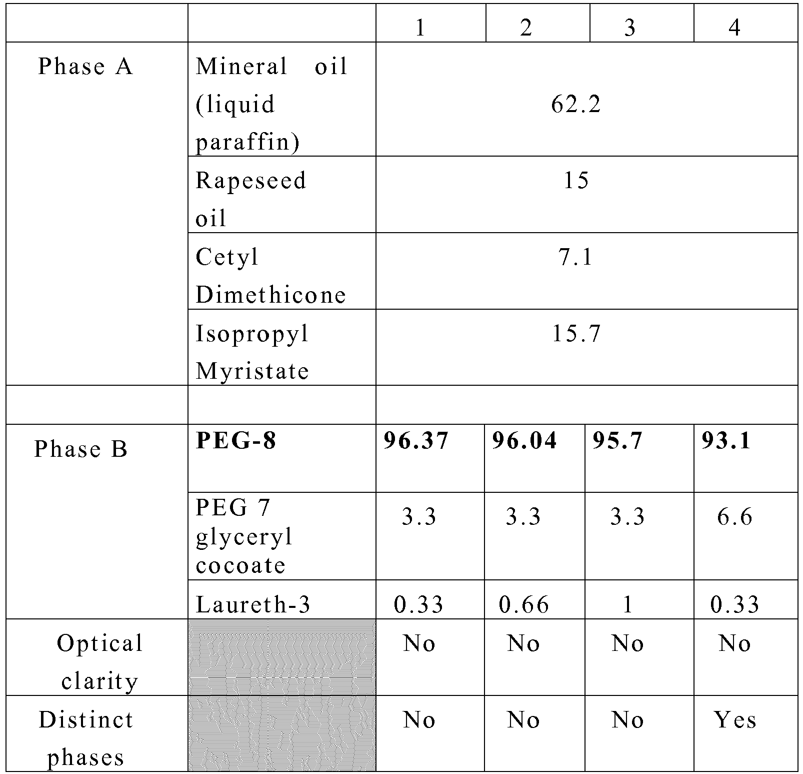

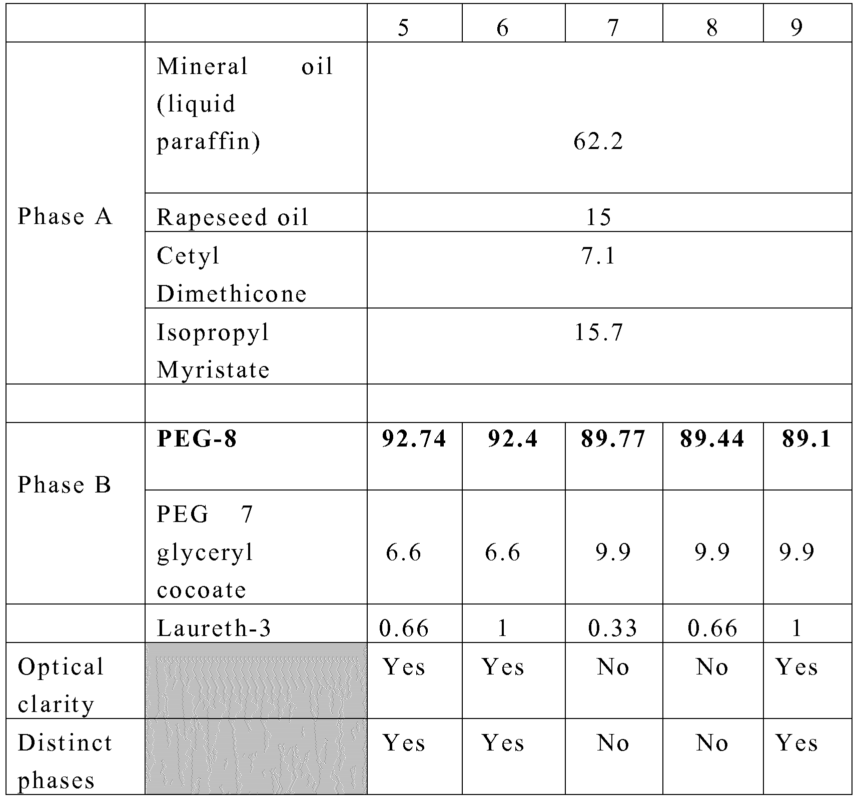

The optical clarity and distinctive phases described above result from a visual observation of the obtained compositions.

### Example 2:

### I. Tested compositions

The following compositions are prepared with the ingredients listed in the table below. The quantities are expressed in grams (g) of active matter.

| | | 1 | 2 | 3 | 4 | 5 | 6 |
|---|---|---|---|---|---|---|---|
| Phase A | Mineral Oil | 62.2 | | | | | |
| | Rapeseed oil | 15 | | | | | |
| | Cetyl dimethicone | 7.1 | | | | | |
| | Isopropyl myristate | 15.7 | | | | | |
| Phase B | Polyol*: | PEG-8 | | Propylene glycol | | Glycerine | |
| | PEG 7 glyceryl cocoate | 6.6 | 9.9 | 6.6 | 9.9 | 6.6 | 9.9 |
| | Laureth-3 | 1 | | | | | |
| Ratio | | 6.6 | 9.9 | 6.6 | 9.9 | 6.6 | 9.9 |
| Optical Clarity | | YES very clear | YES very clear | lower clarity | lower clarity | lower clarity | lower clarity |
| Distinct Phase | | sharp interface | sharp interface | blurred interface | blurred interface | blurred interface | blurred interface |

| | | | | | | | |
|---|---|---|---|---|---|---|---|
| *The concentrations of the polyols used in example 2 correspond to 92,4% and 89,1% by weight in phase (B). | | | | | | | |

### Example 3

The following compositions are prepared with the ingredients listed in the table below. The quantities are expressed in weight percentage of active matter.

| | Composition A (%) |
|---|---|
| Polyethylene glycol (8 OE) | 26.5 |
| PEG-7 glyceryl cocoate | 3 |
| Laureth-3 | 0.3 |
| BRASSICA | |
| CAMPESTRIS (RAPESEED) | 10 |
| SEED OIL | |
| Salicylic acid | 0.1 |
| Cetyl dimethicone (viscosity 15-25 cst - MW : 900) | 3 |
| Phenyl trimethicone (viscosity 20 cst - MW : 372) | 2 |
| Yellow 10 (CI : 47005) | Qs |
| Isopropyl myristate | 11 |
| preservative, fragrance | Qs |
| Mineral oil | Qsp 100 % |

| | Composition B (%) |
|---|---|
| Polyethylene glycol (8 OE) | 26.13 |
| PEG-7 glyceryl cocoate | 3 |
| Laureth-3 | 0.3 |
| BRASSICA | |
| CAMPESTRIS (RAPESEED) | 10 |
| SEED OIL | |
| Cétyl dimethicone (viscosity 15-25 csT - MW : 900) | 3 |
| Phenyl trimethicone (viscosity 20 csT - MW : 372) | 2 |
| Isopropyl myristate | 11 |
| Yellow 6 (CI : 15985) | 0.02 |
| Alpha-tocopherol acetate | 0.1 |
| 2-hydroxy-4-methoxybenzophenone-5-sulfonic-acid | 0.05 |
| Preservative, fragrance | Qs |
| Mineral oil | Qsp 100% |

| | Composition C (%) |
|---|---|
| Polyethylene glycol (8 OE) | 26.13 |
| PEG-7 glyceryl cocoate | 3 |
| Laureth-3 | 0.3 |
| BRASSICA | |
| CAMPESTRIS (RAPESEED) | 21 |
| SEED OIL | |
| Cetyl dimethicone (viscosity 15-25 csT - MW : 900) | 5 |
| Yellow 6 (CI : 15985) | 0.02 |
| Alpha-tocopherol acetate | 0.1 |
| 2-hydroxy-4-methoxybenzophenone-5-sulfonic- acid | 0.05 |
| preservative, fragrance | Qs |
| Mineral oil | Qsp 100% |

The compositions are in the form of bi-phase conditioning oil that could be used as a hair conditioner.

The obtained compositions are optically clear and have two phases that are visually distinctive from each other.

As previously mentioned, the anhydrous composition of the present invention is optically clear.

## Claims

1. Anhydrous composition comprising:
- at least one oil phase (A) comprising at least one oil,
- at least one anhydrous non-oil phase (B) comprising at least one polyol in an amount ranging from 60 to 95% by weight relative to the total weight of the non-oil phase (B).
- at least one non ionic surfactant (i) having an HLB higher than 9
- at least one non ionic surfactant (ii) having an HLB less than or equal to 9 in a quantity of at least 0.2% by weight relative to the weight of the anhydrous composition;
wherein the non ionic surfactant (i) and the non ionic surfactant (ii) are present in the composition in a weight ratio of from 5.5 to 12.5, and
wherein said phases (A) and (B) are visually distinctive from each other.

2. Composition according to claim 1, **characterized in that** the polyols are present in the anhydrous composition in an amount ranging from 10% to 40% by weight, better still from 15% to 35% by weight and even more preferably from 20% to 30% by weight relative to the weight of the composition.

3. Composition according to claim 1 or 2, **characterized in that** the oil used in the oil phase (A) is selected from aliphatic hydrocarbons, plant oils, fatty alcohols, esters of fatty alcohols and/or fatty acids other than animal or plant oils and synthetic glycerides, or mixtures thereof.

4. Composition according to any of claims 1 to 3, **characterized in that** the oil phase (A) comprises at least one aliphatic hydrocarbon and at least one plant oil.

5. Composition according to any one of the preceding claims, **characterized in that** the anhydrous non-oil phase (B) comprises at least a polyol having the following structure: 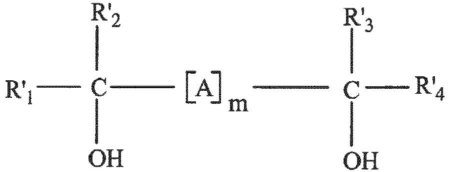
in which R'₁, R'₂, R'₃ and R'₄ denote, independently of each other, a hydrogen atom, a C₁-C₆ alkyl radical or a C₁-C₆ mono- or polyhydroxyalkyl radical,
A denotes a linear or branched alkylene radical containing from 1 to 18 carbon atoms, this radical comprising from 0 to 9 oxygen atoms,
m denotes 0 or 1.

6. Composition according to any one of the preceding claims, **characterized in that** the polyols are chosen from polyethylene glycol such as PEG-8.

7. Composition according to any one of the preceding claims, **characterized in that** the non ionic surfactant (i) having an HLB higher than 9 is chosen from non ionic oxyethylenated surfactant having a number of ethylene oxide higher than 5.

8. Composition according to any one of the preceding claims, **characterized in that** the non ionic surfactant (i) is chosen from oxyethylenated lauryl alcohol containing 12 moles of ethylene oxide (HLB = 14.5), oxyethylenated oleyl alcohol containing 30 moles of ethylene oxide (HLB = 16.6), oxyethylenated oleyl alcohol containing 10 moles of ethylene oxide (HLB = 12.4), polyethylene glycol-7 (PEG-7) glyceryl cocoate (HLB = 11.0) and polyethylene glycol-6 caprylic capric triglyceride (HLB = 13.2).

9. Composition according to any one of the preceding claims, **characterized in that** the non ionic surfactant (ii) having an HLB less than or equal to 9 is chosen from non ionic oxyethylenated surfactant having a number of ethylene oxide lower than or equal to 5.

10. Composition according to any one of the preceding claims, **characterized in that** the non ionic surfactant (ii) is chosen from oxyethylenated decyl alcohol containing 3.5 mole of ethylene oxide (HLB = 8.5), oxyethylenated isostearyl alcohol containing 2 moles of ethylene oxide (HLB = 6.5), oxyethylenated lauryl alcohol containing 3 moles of ethylene oxide (HLB = 8.0), oxyethylenated C₅₀ primary alcohol containing 4 moles of ethylene oxide (HLB = 4).

11. Composition according to any one of the preceding claims, **characterized in that** the weight ratio between the non ionic surfactant (i) and the non ionic surfactant (ii) is ranging from 6 to 12, even better from 6 to 10.

12. Cosmetic process for treating keratin fibers, in particular human keratin fibers such as hair, comprising a step of applying to the said fibers a composition as defined in any of the claims 1 to 11.

## Patentansprüche

1. Wasserfreie Zusammensetzung, umfassend:
- mindestens eine Ölphase (A), die mindestens ein Öl umfasst,
- mindestens eine wasserfreie Nichtölphase (B), die mindestens ein Polyol in einer Menge im Bereich von 60 bis 95 Gew.-%, bezogen auf das Gesamtgewicht der Nichtölphase (B), umfasst,
- mindestens ein nichtionisches Tensid (i) mit einem HLB-Wert von mehr als 9,
- mindestens ein nichtionisches Tensid (ii) mit einem HLB-Wert kleiner oder gleich 9 in einer Menge von mindestens 0,2 Gew.-%, bezogen auf das Gewicht der wasserfreien Zusammensetzung;
wobei das nichtionische Tensid (i) und das nichtionische Tensid (ii) in der Zusammensetzung in einem Gewichtsverhältnis von 5,5 bis 12,5 vorliegen und
wobei die Phasen (A) und (B) visuell voneinander unterscheidbar sind.

2. Zusammensetzung nach Anspruch 1, **dadurch gekennzeichnet, dass** die Polyole in der wasserfreien Zusammensetzung in einer Menge im Bereich von 10 bis 40 Gew.-%, noch besser von 15 bis 35 Gew.-% und noch weiter bevorzugt von 20 bis 30 Gew.-%, bezogen auf das Gewicht der Zusammensetzung, vorliegen.

3. Zusammensetzung nach Anspruch 1 oder 2, **dadurch gekennzeichnet, dass** das in der Ölphase (A) verwendete Öl aus aliphatischen Kohlenwasserstoffen, Pflanzenölen, Fettalkoholen, Estern von Fettalkoholen und/oder Fettsäuren, die von Tier- oder Pflanzenölen und synthetischen Glyceriden verschieden sind, oder Mischungen davon ausgewählt sind.

4. Zusammensetzung nach einem der Ansprüche 1 bis 3, **dadurch gekennzeichnet, dass** die Ölphase (A) mindestens einen aliphatischen Kohlenwasserstoff und mindestens ein Pflanzenöl umfasst.

5. Zusammensetzung nach einem der vorhergehenden Ansprüche, **dadurch gekennzeichnet, dass** die wasserfreie Nichtölphase (B) mindestens ein Polyol mit der folgenden Struktur umfasst: 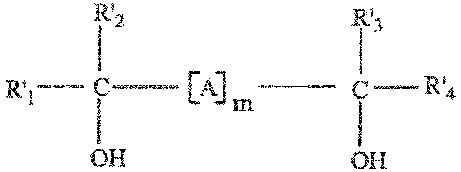
in der R'₁, R'₂, R'₃ und R'₄ unabhängig voneinander für ein Wasserstoffatom, einen C₁-C₆-Alkylrest oder einen C₁-C₆-Mono- oder -Polyhydroxyalkylrest stehen,
A für einen linearen oder verzweigten Alkylenrest mit 1 bis 18 Kohlenstoffatomen steht, wobei dieser Rest 0 bis 9 Sauerstoffatome umfasst,
m für 0 oder 1 steht.

6. Zusammensetzung nach einem der vorhergehenden Ansprüche, **dadurch gekennzeichnet, dass** die Polyole aus Polyethylenglykolen wie PEG-8 ausgewählt sind.

7. Zusammensetzung nach einem der vorhergehenden Ansprüche, **dadurch gekennzeichnet, dass** das nichtionische Tensid (i) mit einem HLB-Wert von mehr als 9 aus nichtionischen oxyethylenierten Tensiden mit einer Ethylenoxid-Zahl von mehr als 5 ausgewählt ist.

8. Zusammensetzung nach einem der vorhergehenden Ansprüche, **dadurch gekennzeichnet, dass** das nichtionische Tensid (i) aus oxyethyleniertem Laurylalkohol mit 12 mol Ethylenoxid (HLB-Wert = 14,5), oxyethyleniertem Oleylalkohol mit 30 mol Ethylenoxid (HLB-Wert = 16,6), oxyethyleniertem Oleylalkohol mit 10 mol Ethylenoxid (HLB-Wert = 12,4) Polyethylenglykol-7(PEG-7)-glycerylcocoat (HLB-Wert = 11,0) und Polyethylenglykol-6-caprylcaprintriglycerid (HLB-Wert = 13,2) ausgewählt ist.

9. Zusammensetzung nach einem der vorhergehenden Ansprüche, **dadurch gekennzeichnet, dass** das nichtionische Tensid (ii) mit einem HLB-Wert kleiner oder gleich 9 aus nichtionischen oxyethylenierten Tensiden mit einer Ethylenoxid-Zahl kleiner oder gleich 5 ausgewählt ist.

10. Zusammensetzung nach einem der vorhergehenden Ansprüche, **dadurch gekennzeichnet, dass** das nichtionische Tensid (ii) aus oxyethyleniertem Decylalkohol mit 3,5 mol Ethylenoxid (HLB-Wert = 8,5), oxyethyleniertem Isostearylalkohol mit 2 mol Ethylenoxid (HLB-Wert = 6,5), oxyethyleniertem Laurylalkohol mit 3 mol Ethylenoxid (HLB-Wert = 8,0), oxyethyleniertem primärem Cso-Alkohol mit 4 mol Ethylenoxid (HLB-Wert = 4) ausgewählt ist.

11. Zusammensetzung nach einem der vorhergehenden Ansprüche, **dadurch gekennzeichnet, dass** das Gewichtsverhältnis zwischen dem nichtionischem Tensid (i) und dem nichtionischem Tensid (ii) im Bereich von 6 bis 12, noch besser von 6 bis 10, liegt.

12. Kosmetisches Verfahren zur Behandlung von Keratinfasern, insbesondere menschlichen Keratinfasern wie Haar, bei dem man auf die Fasern eine Zusammensetzung gemäß einem der Ansprüche 1 bis 11 aufbringt.

## Revendications

1. Composition anhydre comprenant :
- au moins une phase huileuse (A), comprenant au moins une huile,
- au moins une phase non-huileuse anhydre (B), comprenant au moins un polyol en une proportion valant de 60 à 95 % en poids par rapport au poids total de la phase non-huileuse (B),
- au moins un tensioactif non-ionique (i) présentant un rapport hydrophile-lipophile HLB supérieur à 9,
- au moins un tensioactif non-ionique (ii) présentant un rapport hydrophile-lipophile HLB inférieur ou égal à 9, en une proportion d'au moins 0,2 %, en poids calculé par rapport au poids de la composition anhydre,
dans laquelle composition
- le tensioactif non-ionique (i) et le tensioactif non-ionique (ii) se trouvent en un rapport pondéral qui vaut de 5,5 à 12,5,
- et lesdites phases (A) et (B) peuvent être visuellement distinguées l'une de l'autre.

2. Composition selon la revendication 1, **caractérisée en ce que** les polyols se trouvent, dans la composition anhydre, en une proportion valant de 10 à 40 % en poids, de préférence de 15 à 35 % en poids et mieux encore de 20 à 30 % en poids, par rapport au poids de la composition.

3. Composition selon la revendication 1 ou 2, **caractérisée en ce que** l'huile utilisée dans la phase huileuse (A) est choisie parmi les hydrocarbures aliphatiques, les huiles végétales, les alcools gras, les esters d'alcool gras et/ou d'acide gras autres que les huiles animales ou végétales, et les glycérides synthétiques, ou les mélanges de tels corps.

4. Composition selon l'une quelconque des revendications 1 à 3, **caractérisée en ce que** la phase huileuse (A) comprend au moins un hydrocarbure aliphatique et au moins une huile végétale.

5. Composition selon l'une quelconque des revendications précédentes, **caractérisée en ce que** la phase non-huileuse anhydre (B) comprend au moins un polyol présentant la structure suivante : 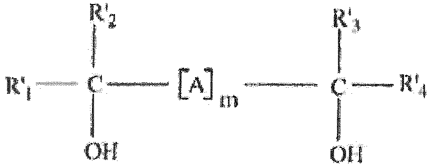 dans laquelle
- R'₁, R'₂, R'₃ et R'₄ représentent chacun, indépendamment les uns des autres, un atome d'hydrogène, un groupe alkyle en C₁-C₆, ou un groupe alkyle en C₁-C₆ monohydroxylé ou polyhydroxylé,
- A représente un groupe alkylène linéaire ou ramifié, comportant de 1 à 18 atomes de carbone, lequel groupe comporte de 0 à 9 atomes d'oxygène,
- et l'indice m vaut 0 ou 1.

6. Composition selon l'une quelconque des revendications précédentes, **caractérisée en ce que** les polyols sont choisis parmi les polyéthylèneglycols, tels que le PEG-8.

7. Composition selon l'une quelconque des revendications précédentes, **caractérisée en ce que** le tensioactif non-ionique (i) présentant un rapport hydrophile - lipophile HLB supérieur à 9 est choisi parmi les tensioactifs non-ioniques éthoxylés comportant plus de 5 motifs dérivés de l'oxyde d'éthylène.

8. Composition selon l'une quelconque des revendications précédentes, **caractérisée en ce que** le tensioactif non-ionique (i) est choisi parmi :
- alcool laurylique éthoxylé avec 12 moles d'oxyde d'éthylène (HLB = 14,5),
- alcool oléylique éthoxylé avec 30 moles d'oxyde d'éthylène (HLB = 16,6),
- alcool oléylique éthoxylé avec 10 moles d'oxyde d'éthylène (HLB = 12,4),
- cocoate de polyéthylèneglycol-7(PEG-7)-glycéryle (HLB = 11.0),
- triglycéride caprique-caprylique-polyéthylèneglycol-6 (HLB = 13,2).

9. Composition selon l'une quelconque des revendications précédentes, **caractérisée en ce que** le tensioactif non-ionique (ii) présentant un rapport hydrophile-lipophile HLB inférieur ou égal à 9 est choisi parmi les tensioactifs non-ioniques éthoxylés comportant un nombre d'oxyde d'éthylène inférieur ou égal à 5.

10. Composition selon l'une quelconque des revendications précédentes, **caractérisée en ce que** le tensioactif non-ionique (ii) est choisi parmi:
- alcool décylique éthoxylé avec 3,5 moles d'oxyde d'éthylène (HLB = 8,5),
- alcool isostéarylique éthoxylé avec 2 moles d'oxyde d'éthylène (HLB = 6,5),
- alcool laurylique éthoxylé avec 3 moles d'oxyde d'éthylène (HLB = 8,0),
- alcool primaire en C₅₀ éthoxylé avec 4 moles d'oxyde d'éthylène (HLB = 4).

11. Composition selon l'une quelconque des revendications précédentes, **caractérisée en ce que** le rapport pondéral entre le tensioactif non-ionique (i) et le tensioactif non-ionique (ii) vaut de 6 à 12, et mieux encore, de 6 à 10.

12. Procédé cosmétique de traitement des fibres kératiniques, en particulier de fibres kératiniques humaines telles que les cheveux, comprenant une étape consistant à appliquer sur lesdites fibres une composition selon l'une quelconque des revendications 1 à 11.
